# EUROPEAN PATENT SPECIFICATION

(11) **EP 1 274 325 B1**
(45) Date of publication and mention of the grant of the patent: **02.11.2006**
(21) Application number: 00977729.3
(22) Date of filing: 24.11.2000
(51) Int. Cl.: A41B 9/12, A41B 11/14, A61F 13/74

(54) **HOSIERY UNDERGARMENT**
STRUMPFHOSE
ARTICLE DE BONNETERIE SERVANT DE SOUS-VETEMENT ET POURVU DE FENTES SUR LES DEUX COTES DE LA FOURCHE

(30) Priority: 20.04.2000 GB 0009655
(43) Date of publication of application: 15.01.2003
(73) Proprietor: Morello, Lucy, Windlesham, Surrey GU20 6HT (GB)
(72) Inventor: Morello, Lucy, Windlesham, Surrey GU20 6HT (GB)
(86) International application number: PCT/GB2000/004487
(87) International publication number: WO 2001/080673

(56) References cited:
- FR-A- 1 522 370
- US-A- 4 301 550
- US-A- 4 589 876
- US-A- 4 807 304
- US-A- 5 711 034

## Description

1.- This invention relates to hosiery undergarments with slits at either side of crotch / gusset.

2.- Traditionally hosiery has been divided into stocking of various lengths and lights in which a body portion is provided with hosiery legs which may extend as far as to cover the toes of the feet.

They are not, however, always suitable in hot weather when many women would prefer to go bear legged. These items of hosiery are normally distinguished from undergarments, such as panties, knickers, etc. in which the bodies of the latter tend to be relatively loose and the leg portions not closely associated with the wearers legs, but finishing just below the buttocks, often creating an unflattering line visible on the outer garment, commonly known as panty line or knicker line.

3.- Garments such as girdles, which have a substantial leg portion beneath the crotch, are usually of such weight that they are not suitable to wear under fashionable close-fitting garments, moreover they cause the wearer to feel restricted in movement and in addition, psychologically their impact is such that many modern women would not consider wearing them.

4.- With fashionable bare legs, close fitting trousers, slacks and shorts, there is a need for a garment which has the advantages of the sculpting yet lightweight structure of a pair of control-tights and yet does not have full length legs as in a pair of tights which cover the feet. However with the lightweight material from which such a garment would be constructed there would tend to be movement or rolling up of the leg portion, which would be both uncomfortable and spoil the line of the outer garment.

5.- Modern sanitary towels used by women of many nationalities are constructed with side flaps designed to wrap around the gusset of underwear worn, with the purpose of holding towel in place. With present styles of tights, girdles and other hosiery garments with a significant leg portion it is not possible to successfully use this fashionable type of sanitary towel with side flaps.

In the prior art, document US-A-4 301 550 cites a solution for elderly or infirm women who must struggle with these garments in order to urinate by providing a garment in which the panty portion comprises a double knit fabric material which by its construction presents overlapping flaps with slit openings to facilitate digital manipulation and expose the opening through which urination can be accomplished without any removal of the pantyhose garment I; document FR-A-1 522 370 presents an interchangeable gusset with curved edges. These two inventions are not configured, however, to permit the use of sanitary wear with side flaps.

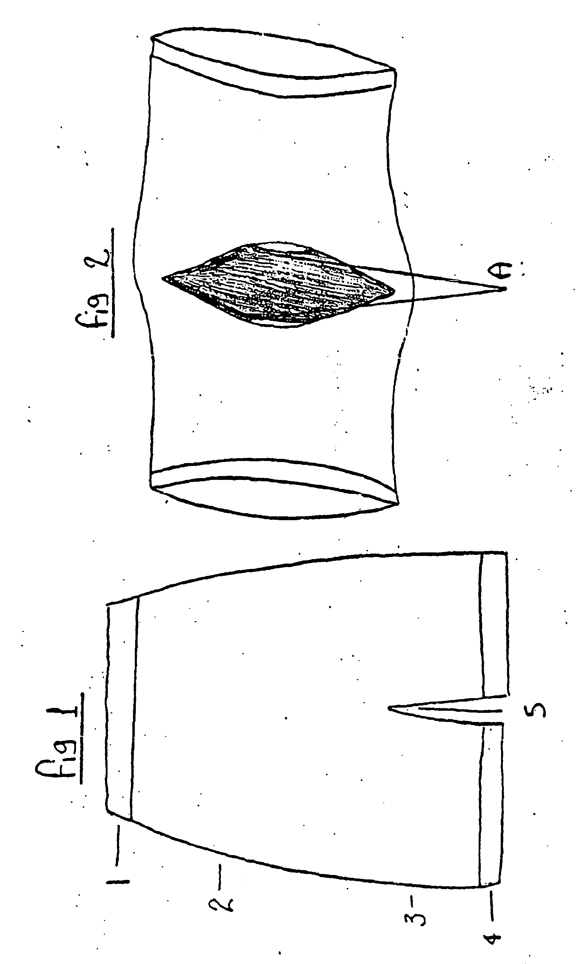

According to the present invention there is provided a garment (Fig.1 illustrates a short version of garment) with a supporting and sculpting body portion (Fig. 1 feature 2) to shape the contours of the body beautifully, of translucent light hosiery weight material, having a significant leg portion (Fig. I features 3 and 4) to avoid visible knicker-lines, yet short enough to be comfortably worn during hot summer days when bare feet or legs are desired, both leg portions (Fig. 1 feature 4) finished with one of either methods described below to avoid such leg portions rising up and with slits on either side of crotch-gusset (Fig.2 feature A) to permit use of modern sanitary towels with side flaps and finished with an upper waist-band (Fig. 1 feature 1) to hold garment in place.

The upper body portion of garment can be finished at different heights of the body as required ie: hip level, waist level or below bust-line level.

By light hosiery weight material is meant is one of the materials well known in the art, for example nylon, rayon, silk, lycra (registered trademark) or blends thereof, usually of a knitted structure, the denier of the fibre and the weight of knitting being appropriate to hosiery type garments ie: relatively transparent or translucent materials, as compared to the more heavy structures of for examples girdles or other rigid garments or materials such as wool. A garment of this nature can have sufficient weight of material and structure to effect control but invisible enough to be worn under close fitting outer garments.

By control, support or sculpting is meant different levels of pressure applied to body of wearer by garment to help shape body of wearer. le: flatten stomach and smooth hips.

The structure and styling of the garment will be such that it is resilient and expandable, so that on donning the garment the body will cause some expansion of the garment, as it is dunned, resulting in a close fit of the garment to the body as compared to looser garments such as panties, knickers and similar garments.

The following describes methods that should be used to hold leg portion in place:

### "methods to hold leg portion in place"

a) Disposed around the inside portion of the leg there could be a sufficient number of areas of increased adhesivity to the leg which can be achieved by use of a polymeric material having a slight adhesive or 'tacky' surface. Material of this nature is well known and is usually silicon rubbers or other elastomeric materials. It is not usually necessary to go to the degree of adhesivity, which would be achieved by a pressure sensitive adhesive. Only a slight degree of tackiness is necessary to hold garment in place. These areas could be of various shapes and distributed around the inner surface of the leg or one or more strips running circumferentially round the inside leg.
b) a band or reinforced structure ie: of a ribbed consistency, similar to bands often used to finish garments at waist height would prevent legs from rising up.

The invention is illustrated in the accompanying drawings in which:
Fig. I is a front view of a short version of the garment according to the invention.
Fig 2 is a frontal view of the crotch area showing slits at each side.

As shown in Fig. I, the garment represents a body of construction similar to the body portion of a pair of conventional tights extending from approximately the waist, down to the legs. The upper portion of leg is of a similar construction to the rest of the garment, the leg portion, which could be of various lengths and extend as far down as the ankles, would be finished with a separate strip of material (4), such as lace or other decorative material sewn later to the leg portion but with some degree of extensibility. Disposed around the internal periphery of this edge portion of the garment may be a series of rings, strips or other shapes of silicon rubber polymeric type materials which are also extensible and which have a slightly tacky feel to them as to ensure adherence to the skin when worn. To use one of the above described methods to finish the lower end of each leg portion is essential to prevent rolling of the garment when worn.

The body portion (Fig. I feature 2) and upper leg portion (Fig. I feature 3) are constructed of a conventional light weight hosiery weight knitted material which stretches on being donned by the wearer, so as to hug the body of the wearer and may have sufficient additional strengthen portions to exercise control over certain portions of the wearer's body. The material used can be a nylon, lycra (registered trade mark) or a mixture of nylon with cotton of other blends thereof.

The crotch area (5) of this under-garment would have a slit (A) on each side of gusset to allow the side flaps of sanitary towels to be exposed outside garment and wrapped around gusset or crotch of the same to prevent movement of sanitary towels. The said slit between the crotch and leg portion can be constructed in various lengths and be finished in well known manners in the industry to avoid fraying of the material.

## Claims

1. Hosiery undergarment **characterised in that** the crotch area (5) has a slit (A) on either side of gusset/crotch to allow the side flaps of sanitary towels to be exposed outside garment and wrapped around gusset or crotch (5) of the same to prevent movement of sanitary towels..

2. Hosiery undergarment according to claim 1 comprising an upper band (1), a body portion (2), a crotch portion (5) with slits(A) on either side of gusset and a significant leg portion (3) which may extend as far as to cover the toes.

## Patentansprüche

1. Wirkwarenunterwäsche, **dadurch gekennzeichnet, dass** der Zwickelbereich (5) auf jeder Seite des Zwickels einen Schlitz (A) aufweist, um es zu gestatten, dass die Seitenlaschen von Damenbinden/Schlüpfereinlagen außerhalb des Kleidungsstücks freigelegt und um den Zwickel (5) desselben herumgewickelt sind, um das Bewegen der einer Damenbinde zu verhindern.

2. Wirkwarenunterwäsche nach Anspruch 1 umfassend ein oberes Band (1), ein Körperteil (2), ein Zwickelteil (5) mit Schlitzen (A) auf jeder Seite des Zwickels und ein wesentliches Beinteil (3), das sich so weit erstrecken kann, dass es die Zehen (4/6) bedeckt.

## Revendications

1. Sous-vêtement (bonneterie) **caractérisé par** des fentes (A) presentes au secteur de fourche (5) pour permettre aux ailerons latéraux des serviettes sanitaires d'être exposées en dehors du vêtement et d'être enroulées autour de la fourche (5) du même pour empêcher le mouvement de la serviette sanitaire.

2. Sous-vêtements selon la revendication 1 peuvent avoir une bande supérieure (1), la partie de corps (2) avec des fentes (A) de chaque côté du secteur de fourche (5) et une partie significative de la jambe (3) cela peuvent se prolonger jusqu'aux orteils(4/6).
